# EUROPEAN PATENT SPECIFICATION

(11) **EP 4 539 857 B1**
(45) Date of publication and mention of the grant of the patent: **11.03.2026**
(21) Application number: 23729446.7
(22) Date of filing: 14.06.2023
(51) Int. Cl.: A61K 31/7076, A61K 9/00, A61P 25/28, A61P 25/00, A61K 47/40, A61K 47/24, A61K 47/36, A61K 47/10, A61K 47/69

(54) **SUBLINGUAL FORMULATION OF CLADRIBINE FOR USE IN THE TREATMENT OF AUTOIMMUNE NEURODEGENERATIVE DISEASES**
SUBLINGUALE FORMULIERUNG VON CLADRIBIN ZUR VERWENDUNG BEI DER BEHANDLUNG VON NEURODEGENERATIVEN AUTOIMMUNERKRANKUNGEN
FORMULATION SUBLINGUALE DE CLADRIBINE POUR UTILISATION DANS LE TRAITEMENT DE MALADIES NEURODÉGÉNÉRATIVES AUTO-IMMUNES

(30) Priority: 15.06.2022 EP 22179104
(43) Date of publication of application: 23.04.2025
(73) Proprietor: Vektor Pharma TF GmbH, 88524 Uttenweiler (DE)
(72) Inventor: WAGNER, Wolfgang, 89250 Senden (DE); HIESTAND, Bianca, 88444 Ummendorf (DE); BECKERT, Thomas, 88447 Warthausen (DE); ANJO, Carlos, 78464 Konstanz (DE); ZABUDKIN, Oleksandr, 76593 Gernsbach (DE); COLOMBO, Angelo, 20861 Brugherio (IT)
(74) Representative: SONN Patentanwälte GmbH & Co KG
(86) International application number: PCT/EP2023/065991
(87) International publication number: WO 2023/242285

(56) References cited:
- WO-A1-2015/023889
- WO-A1-2022/053608
- CA-A1- 2 520 523
- US-A1- 2020 390 837
- US-A1- 2021 267 934
- US-B1- 6 194 395

## Description

### Field of the Invention

The present invention relates to a sublingual pharmaceutical composition comprising the active ingredient cladribine, preferably cladribine incorporated in an 2-hydroxypropyl-*β*-cyclodextrin (HP*β*CD) complex. This active ingredient is uniformly dispersed within the polymeric matrix comprising of at least one hydrophilic polymer within an orodispersible film. This film delivery system suitable for sublingual delivery of the active ingredient cladribine, can be formed during manufacturing in the form of film strips or sheets and subsequently cut into uniform dosage units (orally disintegrating films), each dosage unit being uniform in content.

The pharmaceutical composition may be used as novel medicament, particularly in the treatment of multiple sclerosis (MS), myasthenia gravis (MG) and neuromyelitis optica spectrum disorders (NMOSD).

### Background of the Present Invention

Cladribine (2-CdA), chemically 2-chloro-2'-deoxyadenosine (formula 1) is an antineoplastic agent used in the treatment of some types of leukemias, including hairy cell leukemia (HCL) and B-cell chronic lymphocytic leukemia (CLL).

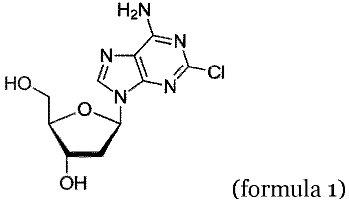

Cladribine shows durable efficacy for reducing frequency and severity of relapses in patients with relapsing-remitting multiple sclerosis (RRMS). Cladribine is highly effective to treat relapsing forms of multiple sclerosis (MS).

Cladribine is a lymphocyte depleting agent comprises a synthetic deoxyadenosine analog with substitution of a hydrogen atom with chlorine at the 2-position of the purine ring. This substitution makes the nucleoside analog resistant to degradation by adenosine deaminase (ADA), an enzyme that metabolizes and clears the naturally occurring deoxynucleosides.

Cladribine enters the cell via nucleoside transporter proteins. Inside the cell, cladribine is activated through three successive phosphorylations, the first of which is catalyzed by the enzyme deoxycytidine kinase (DCK). Activated cladribine can be inactivated through dephosphorylation by the enzyme 5*'*-nucleotidase (5'-NT). Compared with other cells, lymphocytes have a high concentration of DCK and a low concentration of 5*'* -NT, and, therefore, accumulate higher concentrations of the phosphorylated molecule, which becomes trapped inside the cell resulting in a preferential accumulation of phosphorylated, or activated, cladribine.

The treatment with cladribine leads to a preferential and sustained reduction in lymphocytes and monocytes, resulting in long-lasting depletion of CD₄+ and CD8+ T cells and CD₁₉+ B cells, which participate in the development of MS lesions. The key to selectivity - the preferential activity of cladribine to deplete lymphocytes - lies in the high ratio of DCK/₅'-NT activity unique to lymphocytes.

To date cladribine is available only in injectable and oral forms. Until 2017, cladribine was available on the market as final formulation administered only by intravenous injection of saline solutions contained 2mg/ml of cladribine; also off-label subcutaneous administration of cladribine in MS showed effectiveness. Subcutaneous injections of Cladribine are described to be effective in neuromyelitis optica spectrum disorder (NMOSD) *(*Rejdak et al., Eur. J. of Neurology, 2021, 28(9), 3167-3172*)* and in myasthenia gravis (MG) *(*Rejdak et al., Eur. J. of Neurology, 2020, 27(3), 586-589*).*

Off-label cladribine injection presents two problems for subcutaneous injection. First, cladribine is slightly soluble in water, which requires a large volume of material to be injected subcutaneously to achieve the required dose. For example, Rejdak (US 10,350,231 B2) applied 2 mg/mL concentrate for intravenous injections subcutaneously 20 mg (10 mL of solution) 5 consequent days. Eight subcutaneous sites were used, each receiving an injection of 2.5 ml. Such approach is not acceptable for routine clinical practice.

Second, cladribine has limited stability as saline solution. Cladribine's essential property is the instability in an acidic environment. It was demonstrated that at physiological temperature of 37 ° C, the *t*_{*1*/*2*} value of cladribine was determined to be 1.6 hours for pH 2.0 solutions and only 0.37 hours for pH 1.0 solutions.

In order to resolve the application problems Thomas W. Schultz (US 6,194,395 B1) teaches to prepare complex of different types of cyclodextrins (preferably, 2-hydroxypropyl-betacyclodextrin) with cladribine to achieved stable injectable formulation and better solubility. In US 6,194,395 B1 it is noted that employing the cyclodextrin liquid formulations according to patent invention, the solubility of cladribine can be significantly enhanced. The injection volume can be therefore reduced to less than 1 ml (10 mg/mL) per injection. Irritation and pain due to high osmolality or large injection volume is reduced.

In addition, Schultz states that cladribine is significantly more stable at lower pH when combined with cyclodextrins like HP*β*CD. According to the patented invention, cyclodextrins contributed significantly to the stability of cladribine in pH 1.4 solution and maintained more than 80% of the active substance for almost 5 hours and approximately 70% of the active substance for 8 hours, i.e. throughout the experiment, while cladribine dissolved in solution in the absence of cyclodextrins lost about 50% after about 3 hours and after 8 hours only 15% of the active substance remained in solution. Based on the obtained results, the author proposes to use complexes of cladribine with cyclodextrins for the preparation of not only stable liquid formulation but also solid dosage forms of cladribine.

WO 2015/023889 A1 discloses rapidly-dissolving thin film formulations of water-soluble digitalis glycoside for the treatment of congestive heart disease. US 2020/390837 A1 discloses mucosally dissolvable film comprising an emulsion of an extract of cannabis, pullulan, hydroxypropylmethyl cellulose acetate succinate and a flavorant or taste masking agent. US 2021/267934 A1 discloses oral dispersible film compositions wherein an active ingredient with an average particle size of 1 to 20 µ is dispersed within the film. WO 2022/053608 A1 discloses a method for treating an autoimmune disorder in a patient i.a. by optionally administering cladribine orally to the patient. CA 2 520 523 A1 (WO 2004/087101 A2) discloses oral compositions of cladribine and cyclodextrin.

Mavenclad (solid oral dosage form of cladribine) has shown sustained clinical efficacy to treat relapsing forms of multiple sclerosis (MS), to include relapsing-remitting disease and active secondary progressive disease for up to 4 years with a maximum of 20 days of oral treatment over 2 years.

Nicholas S. Bodor has discloses the process for oral formulations of cladribine, in US 7,888,328 *B₂* and US 8,785,415 B2*.* Development of oral cladribine faced the same problems as described above with low solubility in water and stability at low pH. As mentioned above, the essential property of cladribine is the instability in an acidic environment. Because the main site of absorption of drugs administered orally is the low pH stomach's environment, it is difficult to reach the high absorption of acid-sensitive drugs like cladribine.

Bodor advises the approach to oral cladribine formulation using preparation of so-called the cladribine-cyclodextrin inclusion-non-inclusion complex is described in Example 2 of U.S. Patent US 7,888,328 B2, wherein a cladribine-cyclodextrin complex is provided that is an intimate amorphous admixture of an amorphous inclusion complex of cladribine with an amorphous cyclodextrin and amorphous free cladribine associated with amorphous cyclodextrin as a non-inclusion complex, and a pharmaceutical composition comprising said complex, formulated into a solid oral dosage form in form of tablets. Thus, the cyclodextrin itself is amorphous, the inclusion complex with cladribine is amorphous (and is preferably saturated with cladribine) and the free cladribine which forms the non-inclusion complex is amorphous.

Bodor states that the inclusion complex is a complex of cladribine inserted into the hydrophobic cavity of the selected amorphous cyclodextrin, while the non-inclusion/H- bonded complex is amorphous free cladribine loosely hydrogen-bonded to the cyclodextrin. It was estimated that about two-thirds (60 to 70%) of the cladribine will be in the non-inclusion complex, with the remaining one third (30 to 40%) being in the inclusion complex when the product is obtained as exemplified hereinbelow (17% HPβCD solution, 45 to 50°C complexation temperature for about 9 hours); by increasing the percentage of cyclodextrin used and/or manipulating the temperature, products can be readily obtained in which a much greater proportion of the amorphous mixture is in the form of the inclusion complex. In the case of a representative amorphous cyclodextrin, hydroxypropyl-β-cyclodextrin (HPβCD) a cladribine: cyclodextrin weight ratio of from about 1:10 to about 1:16 is appropriate for the exemplified conditions; the ratio is expected to be the same for hydroxypropyl-γ-cyclodextrin under those conditions. The material obtained is characterized by rapid dissolution of the cladribine in aqueous media.

In general, US 7,888,328 B2 addresses the problems of poor bioavailability associated with oral cladribine.

Bioavailability studies are summarized in the EPAR for Mavenclad (EMEA/H/C/004230) including its annexes and by Hermann et al., Clinical Pharmacokinetics. 2019, 58, 283-297.The absolute oral bioavailability in MS subjects is approximately 40%, which is mainly limited by incomplete absorption due to transporter-mediated efflux. In Study IXR-109-09-186 the absolute bioavailability, determined using the ratio of AUC inf oral/IV was 39.1% for the 10 mg oral dose. Interpatient variability, expressed as CV%, was 43.0% for Cₘₐₓ, 29.2% for AUC inf, and 29.8% for AUC t. In Study 25803 the absolute bioavailability was 42.9 %. In Study 93-220 for an oral solution compared to IV solution, mean (SD) bioavailability was 36.7 %.

Examples of factors affecting drug absorption are gastrointestinal motility, gastric emptying rate and the presence of food in the gastrointestinal tract.

The intra-individual variability, as estimated in the population PK analysis, depends on the endpoint (urinary vs. plasma concentration) and the study condition (e.g. i.v. vs. oral). For the CLARITY clinical study, it was estimated to be ~35%. AUC variabilities are ranging between 30% and 35% for the HPβCD tablets.

It is interesting that cladribine, administered as an oral solution without HPβCD, was also rapidly absorbed and calculated that the bioavailability after oral administration of cladribine was 35.3% which is relatively close to bioavailability of HPβCD cladribine tablets. *(Lindemalm et al., BMC Pharmacology 2005, 5(1), 4).*

Low bioavailability could be partially explained by the fact that cladribine is a substrate of BCRP (ABCG2) transporter proteins, which is abundantly expressed in the small intestine.

In *Distribution of breast cancer resistance protein* was also found BCRP mRNA expression was maximal in the duodenum and decreased continuously down to the rectum (terminal ileum 93.7%, ascending colon 75.8%, transverse colon 66.6%, descending colon 62.8%, and sigmoid colon 50.1% compared to duodenum, respectively) and could impact dramatically on cladribine absorption in upper part of gastrointestinal tract. This conclusion was confirmed earlier by Cornelia de Wolf *et al.* in 2008. They show that ABCG2 transports not only the nucleoside monophosphate metabolite of cladribine in analogy with other ABC transporters that can cause resistance to nucleobase and nucleoside analogues but also cladribine itself.
*(*de Wolf et al., Mol Cancer Ther. 2008, 7 (9), 3092-3102*).*

Mavenclad may be also decomposed by PNP (Purine nucleoside phosphorylase) and UP (Uridine phosphorylase) enzymes of intestinal microflora. For evaluation of microflora that could impact on availability of cladribine, the *in vitro* stability of cladribine in bacterial cultures was tested. *Clostridium perfringens* and *Escherichia coli* as well as whole feces rapidly deglycosylated cladribine to Cloroadenine, while *Bacteroides fragilis, Enterococcus faecalis* as well as saliva only degraded cladribine slowly or not at all._Because of deglycosylation of cladribine by colon microflora the bioavailability was only 21% when it was administered rectally.

Low bioavailability and interpatient variability are not only the problems related to cladribine oral administration. Oral formulation like Mavenclad can only be used in those patients who can swallow. Dysphagia is a crucial problem in Multiply Sclerosis. Dysphagia is the medical term for having trouble or difficulty swallowing food or beverages. According to the National Multiple Sclerosis (MS) Society, dysphagia can occur frequently in people living with MS because the disease impairs nerve control of the muscles in the jaw, tongue, and throat that are responsible for chewing and swallowing. The effects of the disease on the brain can cause weakness and coordination issues.

In Levinthal et al.'s study, the prevalence of dysphagia was found to be 21.1% among 218 patients with MS in the United States. (Levinthal et al., Mult. Scler. Int.2013: 319201*).*

In a multicenter study of 1875 Italian patients with MS, 31.3% were found to have dysphagia. Similarly, dysphagic patients had longer disease duration and higher EDSS score than other patients. A recent study found that the prevalence of dysphagia was 45.3% in Turkish patients with MS (Tenekeci et al., Arch. Neuropsychiatr. 55 (2018), 243-247)*.* A further study in 2018 estimated that the general prevalence of dysphagia in those living with MS was about 43% (Aghaz et al., Iran J Neurol. 2018; 17(4), 180-188*).* In a study of Alfonsi et al., the rate of dysphagia in Italian patients with MS even increased to 76.9% (!)(Alfonsi et al., Clin. Neurophysiol. 12 (2013)4, 1638-164*).*

Even more impressive, in the studies of Sales et al. and Fernandes et al., dysphagia was found in, respectively, 58% (Sales et al., Springerplus, 2 (2013), 332*.*) and 90% (!) of Brazilian patients with MS (Fernandes et al., Braz. J. Otorhinolaryngol 79 (2013), 460-465*).*

Mavenclad is designed to be swallowed whole, and crushing or chewing them is not an option. Dysphagia can cause the troubles taking the tablets like Mavenclad. Oral formulation is also unsuitable in patients who are vomiting.

Mavenclad is presumable the subject to first-pass metabolism.

The metabolism of cladribine in isolated perfused rat liver was investigated. The amount of 2-chloroadenine (CAde), the major metabolite of cladribine increased proportionally with time and dose. The first passage effect was approximately 50% (F. Albertioni et al. Eur J Drug Metab Pharmacokinet. Jul-Sep 1995). These data are in contradiction with statement in EPAR for Mavenclad (EMEA/H/C/004230) that any contribution of first pass effect to the intermediate bioavailability of cladribine is negligible.

Main disadvantages associated with the cladribine oral dosage form represent a combination of the narrow absorption window in the upper small intestine, where cladribine becomes the substrate of BCRP (ABCG2) transporter proteins and second degradation by low pH in stomach and by specific enzymes, e.g. PNP, and UP, produced by microbial microflora.

So, it is needed to develop the new advanced formulation of cladribine to overcome the mentioned disadvantages and increase cladribine bioavailability and potentially reduce the daily and total course therapeutic doses.

### Description of the Invention

Therefore, the present invention provides transmucosal delivery compositions in form of sublingual orally dispersible films comprising cladribine as active ingredient, according to claim 1.

The scope of the present invention is defined by the claims.

For the present invention, the term "hydrophilic polymer" refers to polymers containing polar or charged functional groups, rendering them soluble in or swellable by water or aqueous solutions, colloids and suspensions. Hydrophilic polymers may be natural, semisynthetic, and synthetic hydrophilic polymers. Preferred examples of hydrophilic polymers in the compositions according to the present invention are disclosed below.

The used herein term "hydrophilic" refers to the ability of a chemical compound to dissolve in water by having an attractive supramolecular interaction with the water molecule (e.g. hydrogen bonding, dipol-dipol interaction). Hydrophilic can be translated as "water-loving" or "water-liking" and the axiom that "like dissolves like" generally holds true. Thus, hydrophilic substances tend to dissolve in water or other hydrophilic solvent. The hydrophilicity of polymers can be estimated by the solubility of the substance in water.

In case of doubt, the hydrophilicity of the hydrophilic polymers used in the present invention is defined according to their solubility in water. Again, in case of doubts, the hydrophilic polymers used in the present invention have a solubility in water of at least 0.033 g/ml, as defined in Ph. Eur. Chapter 5.11 Characters Section in Monographs - Solubility (as e.g. disclosed in Ph. Eur 11.0, page 805). According to the definition in the Ph. Eur., such polymers are soluble (solubility: 0.033 - 0.1 g/mL), freely soluble (solubility: 0.1 - 1 g/mL) or very soluble (solubility: >1 g/mL) in water.

The solubility parameters in water and the method for measuring of the solubility as described in Ph. Eur. Chapter 5.11 Characters Section in Monographs - Solubility are as follows:

| Solubility | Keyword |
|---|---|
| > 1 g/mL | very soluble |
| 0.1 - 1 g/mL | Freely soluble |
| 0.033 - 0.1 g/mL | Soluble |
| 0.01 - 0.033 g/mL | Sparingly Soluble |
| 0.001 - 0.01 g/mL | Sligthly Soluble |
| 0.0001 - 0.001 g/mL | Very slightly Soluble |
| <0.0001 g/mL | Practically Insoluble |

The method for determination of the solubility of a given polymer as disclosed in the Ph. Eur is as follows:
Weigh 100 mg of finely powdered substance (90) (2.9.12) in a stoppered tube (16 mm in internal diameter and 160 mm long), add 0.1 mL of the solvent and proceed as described under Dissolving Procedure [Dissolving Procedure: Shake vigorously for 1 min and place in a constant temperature device, maintained at a temperature of 25.0 ± 0.5 °C for 15 min. If the substance is not completely dissolved, repeat the shaking for 1 min and place the tube in the constant temperature device for 15 min.]. If the substance is completely dissolved, it is very soluble.

If the substance is not completely dissolved, add 0.9 mL of the solvent and proceed as described under Dissolving Procedure. If the substance is completely dissolved, it is freely soluble. If the substance is not completely dissolved, add 2.0 mL of the solvent and proceed as described under Dissolving Procedure. If the substance is completely dissolved, it is soluble.

If the substance is not completely dissolved, add 7.0 mL of the solvent and proceed as described under Dissolving Procedure. If the substance is completely dissolved, it is sparingly soluble.

If the substance is not completely dissolved, weigh 10 mg of finely powdered substance (90) (2.9.12; also as defined in the Ph. Eur.) in a stoppered tube, add 10.0 mL of the solvent and proceed as described under Dissolving Procedure. If the substance is completely dissolved, it is slightly soluble. If the substance is not completely dissolved, weigh 1 mg of finely powdered substance (90) (2.9.12) in a stoppered tube, add 10.0 mL of the solvent and proceed as described under Dissolving Procedure. If the substance is completely dissolved, it is very slightly soluble.

The structure-forming polymer is forming the polymeric matrix of the thin-film and it is predefining the physical and chemical properties of the polymeric matrix. The structure forming polymer is preferably a non-linear, e.g. branched or cross-linked polymer, with a high degree of polymerization.

The structure forming polymer is selected from starches (modified or unmodified). Additional suitable structure forming polymers may, for example, be selected from the group comprising dextrins, dextrans, gelatins, glycogens, chitosan, xanthan gum, polymerized rosin, alginic acid and alginates, cellulose and cellulose derivatives, and mixtures or combinations thereof. A derivative of a polymer as used in the present invention is a compound that can be derived from a basic structure or compound by a chemical reaction or replacement of a functional group. Accordingly, and as usually referred to in polymer chemistry, a derivative of a given polymer is a polymer, which has the same structure of the polymeric backbone of the given polymer (e.g. for cellulose: glucose units, which are connected by 1,4-glycosidic bonding), but the functional groups of the individual units may be modified, e.g. by substitution (e.g. hydroxyl group is replaced by methoxy, ethoxy, isopropyloxy, etc.). The cellulose derivatives are therefore preferably selected from the group comprising methylcellulose, ethylcellulose, hydroxypropylmethylcellulose, hydroxyethylcelullose, cellulose acetate phthalate, hydroxypropyl methylcellulose phthalate, carboxymethyl ethylcellulose, hydroxypropylmethyl, hydroxypropyl cellulose, cellulose acetate succinate, sodium carboxymethyl cellulose, and mixtures or combinations thereof.

The binding and/or intercalating polymer is interacting with the structure forming polymer and stabilizes and strengthens the matrix formed by the structure forming polymer. The binding/intercalating polymer is preferably a smaller linear polymer with a low degree of polymerization.

The binding and/or intercalating polymer is pullulan. Additional suitable binding and/or intercalating polymers may, for example, be selected from the group comprising maltodextrin, pectins, polylactic acid (PLA), poly-L-lactide (PLLA), poly-D-lactide (PLDA), poly(lactic-co-glycolic acid) (PLGA), poly(methacrylic acid-co-ethyl acrylate), poly(methacrylic acid-co-methyl methacrylate), and mixtures or combinations thereof.

The following synthetic polymers can be applied as structure forming as well as a binding/intercalating polymers dependent on the average molecular weight of the polymers, because of the high variety in the production process of these synthetic polymers. These polymers are selected from the group of polyethyleneoxide, polyethyleneglycol, polyvinylpyrrolidone (PVP), polyvinylalcohols (PVA), polyvinylacetates (PVAc), polyvinyl acetate phthalate and co- or block-copolymers of these polymers. Although there can be certain exceptions due to specific nature of some polymers, for high average molecular weights (MW > 100 000 g/mol) these polymers are typically categorized as structure forming polymers. For lower molecular weights (MW ≤ 100 000 g/mol) the polymers are typically in the category of intercalating/binding polymers.

The combination of at least one of the above-mentioned structure forming polymers with at least one of the above mentioned binding/intercalating polymers is essential for the present invention. The structure forming polymer is pre-defining the properties of the ODFs and by addition of the binding/intercalating polymer the properties, especially the mechanical properties, like disintegration and elasticity, of the thin-film are improved.

However, if hydrophilic polymers are used which can be either structure forming or binding/intercalating dependent on the molecular weight (see description above), the polymers can be from the same functional group. For example, a polyethyleneoxide with MW > 100 000 g/mol can be combined among others with a polyethyleneoxide with MW ≤ 100 000 g/mol within the scope of the invention, since these two polymers are sorted in different categories as described above. If two species of the same polymer category are used as at least one structure forming polymer and at least one binding and/or intercalating polymer according to the present invention, the difference in molecular weight of the two species should be at least 20 %, preferably at least 50 %, especially at least 100 %, as defined from the binding/intercalating polymer species, i.e. the species with the lower MW. If herein reference is made to a MW of a polymer, (in case of doubt) the number average molecular weight is used for defining this MW, i.e. the total weight of polymer divided by the total number of molecules.

As structure forming polymer hydroxypropyl starch is preferred.

The combination of hydroxypropyl starch (as structure forming polymer) and pullulan (as binding/intercalating polymer) is preferred.

According to a preferred embodiment, the at least two hydrophilic polymers are used with a ratio of the first hydrophilic polymer to the second hydrophilic polymer of 95:5 to 5:95 by weight, preferably between 80:20 to 50:50 by weight, more preferably between 70:30 to 60:40 by weight, especially wherein the first hydrophilic polymer is a structure forming polymer and wherein the second hydrophilic polymer is a binding an/or intercalating polymer.

According to a preferred embodiment, cladribine is incorporated in the composition according to the present invention in a 2-hydroxypropyl-*β*-cyclodextrin complex, hydroxypropyl pea starch, pullulan, glycerol and, optionally, soybean lecithin with at least 70% phosphatidylcholine, such as Lipoid S75.

According to a preferred embodiment, cladribine is present in the composition according to the present invention in amounts of up to 0.01% to 50% by weight of the total composition, preferably 0.1% to 20%, more preferably 1% to 10%.

Preferably, the orally disintegrating film composition according to the present invention has a disintegration time of 300 s or less, preferably of 180 s or less, preferably of 120 s or less, especially 60 s or less. Disintegration times of the thin films can be measured in vitro in a solution of phosphate buffered saline (pH = 6.8) at 25 °C to mimic the oral cavity. For the preparation of the dissolution medium 8 g of sodium chloride, 0.2 g of potassium dihydrogen orthophosphate and 2.66 g of disodium hydrogen phosphate dihydrate are dissolved into 1000 mL of demin. water and the pH is adjusted to 6.8 by addition of ortho-phosphoric acid (85%). 25 mL of buffer are filled into a petri dish and are placed onto an orbital shaker. The oral thin film is placed into the petri dish by using a tweezer and the samples are swiveled at a specific rate (75 rpm) to stimulate movement in the solution. The time for complete disintegration is recorded for 3 replicates of each oral thin film example.

The present invention also relates to a transmucosal delivery composition in form of a sublingual orally dispersible film comprising cladribine as active ingredient, wherein cladribine is contained in the composition as a cladribine-cyclodextrin complex, wherein the composition comprises at least two hydrophilic polymers, and wherein the at least hydrophilic polymers represent at least 20% w/w, preferably at least 25% w/w, of the total composition, wherein the at least two hydrophilic polymers comprise at least one structure forming polymer and at least one binding and/or intercalating polymer, wherein the at least one structure forming polymer is selected from starch polymers and wherein the at least one binding and/or intercalating polymer is pullulan.

According to a preferred embodiment, the composition according to the present invention contains cladribine as the single effective ingredient, i.e. only accompanied with further components in the composition which do not have a therapeutic effect on the patients to whom the present compositions are administered, especially with no therapeutic effect for the treatment of MS, MG or NMOSD.

As shown with the present invention, transmucosal cladribine delivery turned out to be a very good solution resolving most of disadvantages related to oral administration.

### Orally disintegrating/dispersible film (ODF) technology

The transmucosal administration of medicinal and therapeutic agents that occur by absorption across the buccal or sublingual mucosa is an attractive alternative to standard oral administration by ingestion into the gastro-intestinal tract, as it can improve the absorption as well as bypass the degradation in the digestive tract. By the novel approach of fast dissolving drug delivery systems, for example orally disintegrating films (also called orally dispersible film, ODF), a beneficial mucosal administration of drugs can be achieved leading to higher control of the actual dosage of the active ingredient and increased patient compliance especially in geriatrics. ODFs are typically the size of a regular postage stamp and disintegrate on a patient's tongue in a matter of seconds for the rapid release of one or more active ingredients. Oral films disintegrate rapidly within seconds when it comes in contact with saliva without the need of water.

The formulation of dissolvable films is customarily facilitated through aqueous polymer matrices that span a wide molecular weight (MW) range enabling the rapid dissolution and fast release of the active pharmaceutical agent upon immediate hydration by saliva in the oral cavity and thereby providing flexibility to achieve certain physical properties.

In comparison to other oral dosage forms, orally disintegrating films have several special advantages such as a rapid disintegrating and dissolution in the oral cavity caused by a larger surface area which improves the onset of action, lower the dosing, and enhance the efficacy and safety profile of the medicament. Since the oral or buccal mucosa being highly vascularized, drugs can be absorbed directly and can enter the systemic circulation without undergoing first-pass hepatic metabolism enabling a reduction of the dose of the active ingredient concomitant with a minimization of associated side-effects. This feature of orally disintegrating films can be exploited in preparing products with improved oral bioavailability of active ingredients.

The sublingual orally dispersible films according to the present invention are provided and manufactured for sublingual administration. This sublingual administration route according to the present invention is to be differentiated against (standard) oral administration. Standard oral administration is performed by applying the API through the mouth by swallowing of the substance, e.g. tablets, capsules, powders and liquids. The API is then absorbed through the gastrointestinal tract (enteral) often resulting in a lower bioavailability. The sublingual administration is a form of transmucosal administration. The API is absorbed directly (not through the gastrointestinal tract = parenteral) through the mucosa in the blood and has therefore usually a better bioavailability. In contrast to such oral administration forms, the films according to the present invention, which can be sublingually administered, are called orodispersible = orally dispersible = orally disintegrating films. In the Ph. Eur. (Chapter - Oromucosal preparations) these dosage forms are defined - in congruence with the use of the term according to the present invention - as: "Orodispersible films are solid oromucosal preparations intended for administration in the mouth, where they disperse rapidly to deliver active substances for a local or systemic effect. They consist of single- or multilayer sheets of suitable materials".

Furthermore, the administered dose of an orally disintegrating film is precisely controlled through the size of the film strip, because the active ingredient is uniformly distributed in the polymer matrix of the film. Moreover, orally disintegrating films are easy in handling transportation and storage and are not as fragile as e.g. orally disintegrating tablets.

Besides, the orally disintegrating film dosage form is very useful for the geriatric and pediatric patients and is highly preferable for patients suffering from dysphagia, repeated emesis, motion sickness, and mental disorders, as they are unable to swallow large quantity of water. Therefore, another practical application for administration via orally disintegrating films include emergency care situations where rapid administration of drugs by non-skilled personnel could be life-saving; in unconscious patients who may have overdosed or experienced a seizure; in elderly dementia patients with dysphagia. The above-described features of orally disintegrating film composition are also very beneficial for the safe and abuse-proof application of drugs.

In summary, the application of cladribine with an orally disintegrating film, which is described in the present invention, is highly desirable and offers numerous advantages compared the oral application in prior art.

The ODF formulation of cladribine allows a precise dosage control and since cladribine is a highly active pharmaceutical ingredient a single therapeutic dose does not exceed 10 mg which can reasonably be incorporated in ODFs. Furthermore, the bioavailability of cladribine is strongly increased by the sublingual application due to cladribine being absorbed and dissolved into the bloodstream through the sublingual mucous membranes absorption. In this regard, the sublingual application also avoids first pass metabolism as cladribine is directly absorbed from the sublingual mucosa and enters into the systemic circulation leading to reduced side effects and dose. As BCRP (ABCG2) transporter proteins are not present in oral mucosa epithelial cells, the mucosal absorption of cladribine is further increased. Thus, a lower daily dose and lower total cumulative dose is required compared to the cladribine formulations of prior art resulting in an improved patient compliance.

Furthermore, the risk of acidic degradation and cladribine degradation caused by specific saliva microflora is reduced by sublingual application comparing with orogastric application, because the saliva has a pH normal range of 6.2-7.6 with 6.7 being the average pH. Resting pH of mouth does not fall below 6.3. In the oral cavity, the pH is maintained near neutrality (6.7-7.3) by saliva.

Finally, since the fast-dissolving films disintegrate rapidly when placed sublingually without the need of water, the cladribine ODF has better acceptability among the dysphagic patients without the risk of chocking. This effect increases the patient compliance, especially for example for treating patients with MS, which are often suffering from dysphagia.

Cladribine formulations for improved oral and transmucosal delivery (Nicolas S Bodor, assignee Ares Trading SA (part of Merck-Serono group)) are disclosed in US 8,623,408 B2 and EP 1 608 343 B1 wherein a composition comprising a cladribine-cyclodextrin complex formulated into a solid oral dosage form or a transmucosal dosage form and a method for enhancing the oral and transmucosal bioavailability of cladribine is described.

It had been found that excess cyclodextrin inhibits the absorption of cladribine from a solid oral dosage form or a transmucosal dosage form comprising a cladribine-cyclodextrin complex, and that a solid oral or a transmucosal dosage form of a saturated cladribine-cyclodextrin complex improves oral and/or transmucosal bioavailability and/or achieves lower interpatient and/or intrapatient variation of the drug.

US 6,194,395 B1 discloses in a specific example an oral composition comprising cladribine, cyclodextrin and i.a. two hydrophilic polymers (microcrystalline cellulose and crospovidone) for use in the treatment of leukemias, such as hairy cell leukemia and chronic myelogenous leukemia, and of disease states and autoimmune disorders, such as multiple sclerosis, autohemolytic anemia, inflammatory bowel disease, rheumatoid arthritis, malignant astrocytoma and the like. The composition is either a solution of cladribine or a solid pharmaceutical oral dosage form, such as tablets, caplets, gelcaps, capsules, chewable tablets, lozenges, or fast dissolving wafers. In the specific example of US 6,194,395 B1, wherein crospovidone and microcrystalline cellulose are contained in a milled extrudate, crospovidone and microcrystalline cellulose are used as excipients, namely as binders, to prepare caramelized pellets as intermediate product for cladribine solid dosage forms in a hot melt-extrusion approach; therefore, using a completely different manufacturing technology as for transmucosal/sublingual formulations. The present orodispersible films avoid all inherent disadvantages of oral route of cladribine administration which can only be used in conscious patients and those patients who can swallow (which is a challenge in view of the fact that the general prevalence of dysphagia in those living with MS is about 43%). Moreover, cladribine is decomposed by gastric acid or by PNP and UP enzymes of intestinal microflora which significantly decreases efficiency of the oral administration via solid compositions, such as tablets, and solution compositions, as disclosed in US 6,194,395 B1. Cladribine is also subject of BCRP (ABCG2) transporter proteins expressed in upper part of intestinum; absorption of cladribine may therefore vary; also due to various factors affecting drug absorption, such as gastrointestinal motility, gastric emptying rate and the presence of food in the gastrointestinal tract. In contrast thereto, sublingual application is characterized by rapid disintegration, improved drug absorption, no degradation at physiological pH in saliva, better acceptability among the dysphagic patients, no risk of chocking accurate dosing and great precision. Although the advantages of sublingual administration are known in principle in the present field, it has nevertheless not been achieved to provide appropriate measures, structures and methods for production to enable sublingual (thin) films comprising a cladribine-cyclodextrin complex which allow an effective treatment of patients, especially MS, MG, or NMOSD patients.

Importantly, US 6,194,395 B1, especially the extrudate in the examples thereof, does not disclose a use of the defined cladribine-cyclodextrin complex which is not trivial for providing sublingual final formulations which require specific manufacturing techniques, also significantly differing from the methods as disclosed in US 7,888,328 B2 wherein the cladribine-cyclodextrin complex has to be isolated first. Also in contrast to the formulations disclosed in US 6,194,395 B1, the present invention enables the manufacturing of novel sublingual dosage forms of cladribine with high accuracy and patient compliance by cladribine-cyclodextrin complex incorporation and homogeneous distribution into a thin film.

Therefore, the films according to the present invention also differ from orally administered products, such as tablets, capsules or wafers by their mechanical properties, such as flexibility or elasticity. A characteristic mechanical parameter for the films according to the present invention is therefore the folding endurance. Accordingly, the folding endurance value of the orodispersible films according to the present invention should be at least 10, preferably 50, preferably 100, more preferably 300.

The flexibility and elasticity of the film can be determined by the folding endurance value. Examining the number of folds gives an indication of ODF brittleness and is important for their storage stability and administration without being broken and therefore also for the patient compliance of the product. The flexibility and elasticity of an ODF can be determined by the folding endurance of a film. For the folding endurance test either the ODFs or a sample of the film with rectangular shape and an area of 10 cm² (e.g. 2 x 5 cm) is investigated. The sample is repeatedly folded manually at the same folded edge an angle of 180° until the film breaks. The account of folds until the film breaks is the folding endurance value. This method for determining the folding endurance is also disclosed in Mahesh et al. (Curr. Drug. Deliv. 7 (2010), 21 -27) and Takeuchi et al. (Int. J. Pharmac. 589 (2020), 119876). Commonly > 300 folds per film are called as excellent flexibility.

US 8,623,408 claims a pharmaceutical composition comprising a saturated cladribine-cyclodextrin complex formulated into a solid oral dosage form, said composition being substantially free of cyclodextrin in excess of the minimum amount required to maximize the amount of cladribine in the complex, or to maintain substantially all of the cladribine in the complex, wherein the cyclodextrin is γ-cyclodextrin, wherein the weight ratio of cladribine to γ-cyclodextrin is from about 1:35 to about 1:50 and wherein the complex comprises a 1:2 molar ratio cladribine:y-cyclodextrin complex.

In US 8,623,408 description part Nicolas S. Bodor differentiates explicitly the transmucosal delivery methods and forms and oral dosage forms and teaches that transmucosal forms do not include the methods and forms for oral use, which are intended to be swallowed and are simply called oral dosage forms.

According to Bodor when the transmucosal form is a liquid, it can be obtained by dissolving the saturated complex in a minimum amount of water, for example 500 mg of the saturated complex with HPβCD in 0.5 ml water (50% w/w solution), or 500 mg of the saturated γCD complex in 1.0 ml of water.

A few drops of such a solution can be inserted into the buccal cavity and retained there for about 2 minutes to allow for absorption through the buccal mucosa. Bodor describes that solid transmucosal dosage forms are generally preferred over liquid forms but doesn't provide any experimental data related to solid transmucosal composition.

Instead, it is mentioned that oral or mucosal absorption may be further facilitated by the addition of various excipients and additives to increase solubility or to enhance penetration, such as by the modification of the microenvironment, or by the addition of mucoadhesive excipients to improve contact between the delivery system and the mucosal tissue.

According to the present invention, a wide range of cyclodextrins can be used for cladribine complexation including γ-cyclodextrin; hydroxyalkyl, e.g. hydroxyethyl or hydroxypropyl, derivatives of β- and γ-cyclodextrin; carboxyalkyl, e.g. carboxymethyl or carboxyethyl, derivatives of β- or γ-cyclodextrin; β-cyclodextrin sulfobutyl ether; dimethyl-β-cyclodextrin; and randomly methylated β-cyclodextrin, but experimental data are only related to γ-cyclodextrin and 2-hydroxypropyl-β-cyclodextrin (HPβCD) complexes.

Bodor also provides long list of various carriers could be used for preparation of so called "buccal dosage unit" but doesn't provide any qualitative and quantitative composition of buccal cladribine formulation.

Examples of provided polymeric carriers include acrylic acid polymers and copolymers, e.g., those known as "carbomers" for example, Carbopol^{®}. Other suitable polymers include hydrolyzed polyvinyl alcohol, polyethylene oxides (e.g., Sentry Polyox^{®}), polyacrylates (e.g., Gantrez^{®}), vinyl polymers and copolymers, polyvinylpyrrolidone, dextran, guar gum, pectins, starches, and cellulosic polymers such as hydroxypropyl methylcellulose (e.g., Methocel^{®}), hydroxypropyl cellulose (e.g., Klucel^{®}), hydroxypropyl cellulose ethers, hydroxyethyl cellulose, sodium carboxymethyl cellulose, methyl cellulose, ethyl cellulose, cellulose acetate phthalate, cellulose acetate butyrate, and the like.

Bodor states also that any polymeric carriers can be used that are pharmaceutically acceptable, provide both a suitable degree of adhesion and the desired drug release profile, and are compatible with the cladribine to be administered and any other components that may be present in the buccal dosage unit.

It is also stated the buccal carrier can comprise a polymer having sufficient tack to ensure that the dosage unit adheres to the buccal mucosa for the necessary time period, i.e., the time period during which the cladribine is to be delivered to the buccal mucosa. Bodor summarized that generally the polymeric carriers comprise hydrophilic (water-soluble and water-swellable) polymers that adhere to the wet surface of the buccal mucosa.

It is understandable from the detailed description of US 8,623,408 that all provided carriers in combination with cladribine-cyclodextrin complex are applicable for preparation of buccal adherence solid formulation.

In US 8,623,408 or US 6,194,395 B1, there is not any information provided regarding the sublingual formulation or specifically for sublingual application by thin orally dispersible films.

In contrast thereto, the present invention describes a sublingual pharmaceutical composition, in the form of an orodispersible film, comprising the active ingredient cladribine, wherein cladribine is contained in the composition as a cladribine-cyclodextrin complex, preferably cladribine incorporated in a 2-hydroxypropyl-*β*-cyclodextrin (HP*β*CD) complex. This active ingredient is uniformly dispersed within the polymeric matrix comprising of at least one bioedible water-soluble polymer within an orodispersible film. The incorporation of the active ingredient in the polymeric matrix of the orally disintegrating film is prerequisite for a safe and controlled sublingual application of cladribine. The pharmaceutical composition may be used as a medicament, particularly in the treatment of multiple sclerosis (MS), myasthenia gravis (MG), or neuromyelitis optica spectrum disorders (NMOSD). Therefore, the present invention also relates to the use of the composition according to the present invention for the manufacturing of a medicament for the treatment of MS, MG, or NMOSD. The present invention also relates to a method of treatment of a patient suffering from multiple sclerosis (MS), myasthenia gravis (MG), or neuromyelitis optica spectrum disorders (NMOSD), wherein an effective amount of a composition according to the present invention is administered to a patient in need thereof.

The formulation of cladribine as ODF according to the present invention offers several advantages, such as high bioavailability, lower dosage, increased patient compliance and reduced side-effects.

As will be understood by one skilled in the art, for any and all purposes, such as in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art, all language such as "up to," "at least," and the like include the number recited and refer to ranges which can be subsequently broken down into subranges as discussed above. Finally, as will be understood by one skilled in the art, a range includes each individual member. Thus, for example, a group having 1-3 units refers to groups having 1, 2, or 3 units.

As used herein, the terms "orally disintegrating film", "orodispersible film", "orally dispersible film", "oral film", "orally disintegrating film strip", "film strip", "thin-film" and "film" refer to sheets of variable dimensions comprising a polymeric carrier matrix, and having any shape, including rectangular, square, hexagonal, circular or other desired shape. The films described herein are typically thin films with thickness that can range from 10 microns to 800 microns, preferably from 80 to 600 microns, more preferably from 100 to 300 microns, but may be any desired thickness and size so long as they can be placed comfortably into the oral cavity of the user. Films are a single layer and are typically inherently flexible to enable rapid dissolution in the mouth, permeation through the oral mucosa and entry into the bloodstream via capillaries.

### Components of the ODF

Generally, the present invention is related to an orally disintegrating film having the active agent (cladribine) being distributed within a polymeric matrix, comprising of a hydrophilic film forming polymer or a combination of two or more film-forming polymers. The cladribine is in the form of a cladribine-cyclodextrin complex in the formulation. Furthermore, surfactants, plasticizers and or other additives known in the field may be used for the manufacturing process to achieve orally disintegrating films with the desired properties.

### Active agent according to the present invention

Cladribine is used as active ingredient in the present invention. The chemical structure of cladribine, chemically 2-chloro-2'-deoxyadenosine, is shown in formula 1.

The cyclodextrins within the scope of this invention are amorphous derivatives of the natural cyclodextrins α-, β- or γ-cyclodextrin wherein one or more of the hydroxy groups are substituted, for example, by alkyl, hydroxyalkyl, carboxyalkyl, alkylcarbonyl, carboxyalkoxyalkyl, alkvlcarbonyloxyalkyl, alkoxycarbonylalkyl or hydroxy-(mono or polyalkoxy) alkyl groups; and wherein each alkyl or alkylene moiety preferably contains up to six carbons. Although commonly referred to as a single entity, an amorphous cyclodextrin is actually a mixture of many different entities, since the substituent groups can be located on various hydroxyls of the basic cyclodextrin structure. This in turn results in the amorphous nature of these cyclodextrins, which is indeed well known. Moreover, these cyclodextrins can be obtained in varying degrees of substitution, for example from 1 to 14, preferably from 4 to 7; the degree of substitution is the approximate average number of substituent groups on the cyclodextrin molecule, for example, the approximate number of hydroxypropyl groups in the case of the hydroxypropyl-β-cyclodextrin molecule, and all such variations are within the it of this invention. Substituted amorphous cyclodextrins, which can be used in the invention, include polyethers. Other cyclodextrins contemplated for use herein included glucosyl-β-cyclodextrin and maltosyl-β-cyclodextrin. Of particular utility for the complexation are dimethyl-β-cyclodextrin, randomly methylated β-cyclodextrin and polyethers such as hydroxypropyl-β-cyclodextrin, hydroxyethyl-β-cyclodextrin, hydroxypropyl-γ-cyclodextrin, and hydroxyethyl-γ-cyclodextrin, carboxymethyl-β-cyclodextrin, as well as sulfobutyl ethers, especially sulfobutyl-β-cyclodextrin. In addition to simple cyclodextrins, branched cyclodextrins and cyclodextrin polymers may also be used.

Preferably, 2-hydroxypropyl-β-cyclodextrin is used for complexation of the active ingredient to form the cladribine - 2-hydroxypropyl-β-cyclodextrin (HP*β*CD) complex.

The respective cladribine complex may be prepared according to literature know procedures (e.g. US 8,623,408 B2/EP 1 608 343 B1). The complex can be prepared using either the selected amorphous cyclodextrin, such as hydroxypropyl-β-cyclodextrin (HP*β*CD) or hydroxypropyl-cyclodextrin, or cladribine as the fixed variable to which an excess of the other is added. Typically, cladribine is added to an aqueous solution having a known concentration of amorphous cyclodextrin under known conditions to promote complex formation. Generally, the complexation is conducted with heating, for example at 30 - 60 °C for a significant period of time, e.g., at least 1-24 hours, preferably 1-6 h. The excess precipitated cladribine can then be removed and the cladribine concentration is subsequently measured. The complex can either be used directly for further formulation, preferably in a one-pot reaction, or the suspension of the complex can be transferred into another reaction vessel, or it can be isolated prior for further use. The complex is then homogenously distributed within the polymeric matrix to ensure the homogenous distribution of the active agent within the orally disintegrating film.

The active ingredient is applied for the treatment of any cladribine-responsive disease. Several disease states responsive to cladribine are well-documented in the literature (see also uses of thin film below). For any target disease state, an effective amount of the optimized cladribine-cyclodextrin complex is used (e.g., an amount effective for the treatment of multiple sclerosis, rheumatoid arthritis, or leukemia etc.).

According to a preferred embodiment, the composition according to the present invention contains cladribine as the single effective ingredient. The term "effective ingredient" relates to any specifically effective drug for which a market authorization is present, especially in the European Union or in the US. This means that the present composition does not contain a further effective ingredient, especially no effective ingredient which is authorized to be administered to patients for the treatment of MS, MG or NMOSD. The "single effective ingredient" language does not exclude the use of substances in this preferred composition as stabilizers, (co-)surfactants, film forming components, plasticizers or further additives, even if such substances may have a beneficial effect on health (as e.g. antioxidants or vitamins).

### Dosage of the active ingredient

The desired dosage of the active ingredient in the ODF is defined by the active dosage needed for the treatment of the specific disease. The amount of active per unit area is determined by the uniform distribution of the film. For example, when the films are cut into individual dosage forms, the amount of the active in the dosage form can be known with a great deal of accuracy. This is achieved because the amount of the active in a given area is substantially identical to the amount of active in an area of the same dimensions in another part of the film. The accuracy in dosage is particularly advantageous when the active is a medicament, i.e. a drug.

Drug loading into film may be up to about 50% by weight of the film, and often up to 1% to 10% by dry weight. The amount of drug in each film can be calculated before or after shaping the individual films or ingestible unit into the size and shape of the dosage form. The amount of drug lost during processing can also be taken into account in order to design the ingestible units and select the appropriate number of sheets to arrive at the predetermined dose. The films are capable of providing an accurate dosage amount (determined by the size of the film and concentration of the active in the original polymer/water combination) regardless of whether the required dosage is high or extremely low.

### Film Forming Polymer

The polymeric matrix of the orally disintegrating film composition is formed by a combination of at least two hydrophilic polymers comprising starch-based polymers (modified or unmodified), and pullulan.

The film-forming polymer comprises starches (modified or unmodified). Additional film-forming polymers may, for example, be selected from the group comprising dextrins, dextrans, gelatins, glycogens, chitosan, xanthan gum, polymerized rosin, alginic acid and alginates, cellulose and cellulose derivatives (modified or unmodified), comprising methylcellulose, ethylcellulose, hydroxypropylmethylcellulose, hydroxyethylcelullose, cellulose acetate phthalate, hydroxypropyl methylcellulose phthalate, carboxymethyl ethylcellulose, hydroxypropylmethyl, hydroxypropyl cellulose, cellulose acetate succinate, sodium carboxymethyl cellulose, maltodextrin, pectins, polylactic acid (PLA), poly-L-lactide (PLLA), poly-D-lactide (PLDA), poly(lactic-co-glycolic acid) (PLGA), poly(methacrylic acid-co-ethyl acrylate), poly(methacrylic acid-co-methyl methacrylate), polyethyleneoxide, polyethyleneglycol, polyvinylpyrrolidone (PVP), polyvinylalcohols (PVA), polyvinylacetates (PVAc), polyvinyl acetate phthalate and mixtures or combinations thereof, preferably modified starch and pullulan.

The source of the starch may vary and include acorn, arrowroot, arracacha, bananas, barley, beans, breadfruit, buckwheat, canna, cassava, chestnut, chickpea, corn, favas, katakuri, kudzu, lentil, malanga, millet, oat, oca, pea, potato, rice, rye, sago, sorghum, sweet potato, taro, tapioca, water chestnut, wheat, yams and combinations thereof. These starches can be gelatinized, unmodified or modified, including chemically and synthetically modified starch, selected for example from the group of Dextrin, acid-treated starch, alkaline-treated starch, bleached starch, oxidized starch, enzyme-treated starch, monostarch phosphate, distarch phosphate, phosphated distarch phosphate, acetylated distarch phosphate, starch acetate, acetylated distarch adipate, hydroxyethyl starch, hydroxypropyl starch, hydroxypropyl distarch phosphate, hydroxypropyl distarch glycerol, starch sodium octenyl succinate, oxidized starch.

According to a specifically preferred embodiment, the orally disintegrating film composition according to the present invention comprises hydroxypropyl pea starch and pullulan as film-forming polymers.

### Surfactant

For a uniform distribution of the active ingredient, at least one surfactant and/or cosurfactant may be used. The surfactant may include ionic surfactants including anionic and/or cationic surfactants such as sodium dodecyl sulfate (SDS), sodium lauryl sulfate (SLS), benzalkonium chloride, benzthonium chloride, benzyldimethyldodecyl ammonium bromide (BDDAB), and non-ionic surfactants such as polysorbate 80, sorbitan monooleate, lecithins, glycolipids, fatty alcohols, fatty acids, esters of fatty alcohols and fatty acids, sorbitan esters, polyols such as polysorbates, sorbitans, long-chain aliphatic acids that can be saturated or unsaturated and having more than 6 carbons, such as stearic acid, lauric acid, oleic acid, linoleic acid, PEG-40 hydrogenated castor oil, sodium deoxycholate, poloxamers, bile salts such as sodium taurocholate, phospholipids, phosphatidylcholines, phosphatidylethanolamines, phosphatidylinositols, phosphatidylserines, phosphatidic acids, polyglycolized glycerides, polyoxyethylene glycerides, polyethylene glycol-fatty acid esters, polyethylene glycol glycerol fatty acid esters, transesterification products of oils and alcohols, polyglycerized fatty acids, glycerol fatty acid esters, polyglycerol fatty acid esters, propylene glycol fatty acid esters, mono and diglycerides, polyoxyethylene-polyoxypropylene block copolymers, polyethylene glycol sorbitan fatty acid esters, sorbitan fatty acid esters derivatives thereof, and combinations thereof. Other suitable surfactants could also be used, and preferably, low molecular weight surfactants are advantageous.

The amounts used of such surfactants can range from 0.1 wt% to 20 wt%, and more preferably between 0.5 wt% to 5 wt% of total, for example, 1 wt% of total, although it is possible to use surfactants that are out of this range.

### Plasticizers

A plasticizer may be used in the formulation of the oral disintegrating film strip. The plasticizer should be a food-grade or pharmaceutical-grade compound, for example one or more of the include low molecular weight polyols such as glycerol, propylene glycol, polyethylene glycols, monosaccharides such as xylitol, erythritol, mannitol, sorbitol; disaccharides such as sucrose, lactose, and maltose; oligosaccharides such as glycogen, inulin, and dextrins such as maltodextrin and similar compounds; citrate derivatives such as citric acid, citrate, tributyl citrate, triethyl citrate, acetyl citrate, citrate ester, triacetin, castor oil, medium-chain triglycerides (MCT) of fatty acids (also known as MCT oil), and various plant oils of low viscosity such as soybean oil, canola oil, corn oil and similar oils, mineral oil, miglyol, derivatives thereof, and combinations thereof. The amounts used of such plasticizer can range from 0.5 wt% to 25 wt%, and more preferably between 5 wt% to 20 wt% of total, for example, 15 wt% to 20 wt% of total, although it is possible to use plasticizers that are out of this.

### Coloring/Flavoring/ Sweetening/ Taste masking/ Stabilizing and Thickening Agents

Coloring agents may also be present in the oral disintegrating film and could include titanium dioxide, and dyes suitable for food such as those known as F.D. & C. dyes and natural coloring agents such as grape skin extract, beet red powder, beta-carotene, annatto, carmine, turmeric, paprika, etc. The colorings agent, if present in the film, may range from 0 to 5 wt% of the total composition, for example, 1 wt% of the total composition.

Flavoring agents may also be present in the oral disintegrating film and could include synthetic flavor oils and flavoring aromatics and/or natural oils, extracts from plants, leaves, flowers, fruits and so forth and combinations thereof. These may include cinnamon oil, oil of wintergreen, peppermint oils, clove oil, bay oil, anise oil, eucalyptus, thyme oil, cedar leave oil, oil of nutmeg, oil of sage, oil of bitter almonds and cassia oil. Also useful as flavors are vanilla, citrus oil, including lemon, orange, grape, lime and grapefruit, and fruit essences, including apple, pear, peach, strawberry, raspberry, cherry, plum, pineapple, apricot and so forth. Other flavors which are used in the field include commercially available orange, grape, cherry and bubble gum flavors and mixtures thereof. Flavor acids, such as citric acid, malic acid, tartaric acid, lactic acid, and ascorbic acid, may also be used to provide acidic tastes. Flavors may be present in an amount ranging from 0.5% to 10.0% by weight based upon the weight of the composition.

Sweetening or taste masking agents may also be present in the oral disintegrating film and could include natural sweeteners, for example, sucrose, stevia, corn syrup, honey, maple syrup, erythritol, maltitol, mannitol, dextrose, fructose, glucose, xylitol, sorbitol, isomalt, and the like, or combinations thereof; and artificial sweeteners, for example, aspartame, sucralose, acesulfame potassium, saccharin, saccharin cyclamate, cyclodextrin derivatives, and the like, or combinations thereof. The amount of the one or more sweetener agents may range from 0 to 2.5 weight percent, for example, about 1 wt% based on the total weight of the composition.

The oral disintegrating film strip may also include one or more of a permeation or permeability enhancer, a bitter blocker, a filler, an effervescent agent, an anti-oxidant, a disintegrating agent, a pH modifying agent, a buffer, a complexing agent, a bioadhesive, a sheet adhesive, an emulsifying agent, a crystallization inhibitor, a preservative, a unique identifying agent such as a UV active fluorophore, and an antimicrobial.

An anti-oxidant may also be added to the film to prevent the degradation of an active, especially where the active is oxygen- or photosensitive.

### Preparation method of the ODFs

First, the complex of cladribine and the cyclodextrin is formed. The formation of the complex is highly beneficial, because the complex of cladribine with cyclodextrin has a much higher solubility in water and an increased stability in aqueous media compared to the non-complexed cladribine.

For preparation of the cladribine-cyclodextrin complex, the cladribine is mixed with the cyclodextrin, preferably 2-hydroxypropyl-β-cyclodextrin (Kleptose HPB), in water by stirring at 100 - 1000 rpm, e.g. 300 rpm, at a temperature of 20-60 °C, preferably 40-50 °C. The target mixing time is 1-24 h, preferably 2-4 h, to ensure the complex formation and a homogenous distribution. The molar ratio of cladribine to the cyclodextrin is ranging from 10:1 to 1:10, preferably 1:0.5 to 1:9, more preferred 1:1 to 1:9, especially 1:1 to 1:3, and is dependent on the used cyclodextrin. For 2-hydroxypropyl-β-cyclodextrin a molar ratio cladribine and cyclodextrin of 1:1 to 1:3 is preferred. This molar ratio conforms to a weight ratio of the components cladribine and HP*β*CD of 1:5 to 1:15.

Accordingly, the present invention also relates to a method for producing an orally disintegrating film composition and/or a transmucosal delivery composition in form of a sublingual orally dispersible film according to the present invention, wherein the cladribine or cladribine-cyclodextrin complex is mixed with the film-forming polymers by stirring with at least 100 rpm for at least 10 min at a temperature of at least 30°C.

Preferably, the mixture is degassed after mixing for at least 1 h, preferably for at least 6 h. According to a preferred embodiment of the present method, the mixture is casted and dried at a temperature of at least 60°C, preferably at a temperature of 65°C to 100°C. Preferably, the mixture is casted and dried to a film with a thickness of 10 to 800 µm, more preferred of 50 to 500 µm, especially of 100 to 200 µm.

The cladribine-cyclodextrin complex can be produced and isolated before further use, but preferably the sublingual film composition comprising the cladribine-cyclodextrin complex according to the present invention is mixed directly after preparation with the other component of the ODF in a one-pot procedure, excluding intermediate isolation and providing a very uniform distribution of active ingredient potency across the film surface, because the active ingredient is uniformly distributed within the polymeric matrix. Thus, the thin film according to the present invention has a low relative standard deviation (RSD) of the concentration of active ingredient. The RSD of the assay of the active ingredient should be below 5%, preferably <2% and more preferably <1%.

This feature of the ODF is resulting in more accurate dosing and thereby resulting in more consistent therapeutic efficacy, as compared to method of cladribine solid formulation designed for oral administration, such as tablets, capsules, or fast dissolving wafers.

In another reaction vessel the surfactants, the plasticizer and all other water-soluble excipient and additives (e.g. flavoring or coloring agents, preservatives etc.) were mixed in water at (20-60 °C) until all components are homogeneously dispersed to form the hydrophilic phase. These two reaction mixtures were mixed and homogenized at 20-60 °C by stirring or applying mechanical agitation at high shear rates for example by using an Ultra-Turrax^{®}. After that the hydrophilic polymer or the mixture of different hydrophilic polymers (e.g. modified starch and pullulan in the respective ratios) are added and the mixture is homogenized by stirring at 20-60 °C by stirring or applying mechanical agitation at high shear rates for example by using an Ultra-Turrax^{®}.

As an alternative preparation method of the coating mass, a one-pot process can be applied for the formation of the complex of the active ingredient and for the mixing of the components.

The resulting dispersion is optionally degassed by stirring at room temperature or at 30 - 50 °C for 1-24 h or by stirring briefly under reduced pressure. After that, a sufficient amount of water is added to exactly adjust the desired solid content (10-80 wt% w/w) and viscosity (100-10000 cP) of the final coating mass.

The stable dispersion was then preferably cast to form an oral disintegrating film. The oral disintegrating film was formed by placing the stable dispersion onto a clean receiving substrate or the like to form the oral disintegrating film. The stable dispersion may be dispensed from a suitable apparatus equipped with a doctor blade as would be known in the art, such as a dispensing apparatus, onto the receiving substrate. The stable dispersion may be poured, injected, or otherwise deposited onto the receiving surface by any suitable process or means. The receiving surface can be of any suitable type, such as a tray on a conveyor belt, or a conveyor belt itself. Otherwise, the release liner itself can also be constantly moved by a drive roller to guarantee its continuous motion during the casting process.

The cast stable dispersion was dried by being subjected to preferably an infra-red heater system or alternatively heat or hot air in order to form the oral disintegrating films. The apparatus can move the receiving surface through e.g., a convention style oven, or alternatively, the drying can be done in a batch process. For example, the drying can be done with a substrate temperature set to 40-110 °C, for example to about 80 °C, with an infrared-heater system. Preferably, the mixture is casted and dried at a temperature of at least 60°C, preferably at a temperature of 65°C to 100°C. In a typical embodiment, drying was performed for between 1 and 15 minutes. After the oral disintegrating film has been dried, it would have a uniform thickness in the range of 20 to 800 microns. According to a preferred embodiment, the mixture is casted and dried to a film with a thickness of 10 to 800 µm, preferably of 200 to 700 µm, especially of 100 to 300 µm.

The film can be cut into any desirable shape and size, e.g. by a knife/scalpel or by a LASER cutting process. Alternatively, the stable dispersion may be cast into forms of the desired size and shape, which eliminated the cutting step.

One skilled in the art will appreciate that, for this and other processes and methods disclosed herein, the functions performed in the processes and methods may be implemented in differing order. Furthermore, the outlined steps and operations are only provided as examples, and some of the steps and operations may be optional, combined into fewer steps and operations, or expanded into additional steps and operations without detracting from the essence of the disclosed embodiments.

### Uses of thin films

The oral disintegrating film strips can be used to treat relapsing forms of multiple sclerosis, to include relapsing-remitting disease and active secondary progressive disease, as well as primary progressive multiply sclerosis. ODF can be used for treatment other neurodegenerative diseases having autoimmune nature as myasthenia gravis and neuromyelitis optica spectrum disorders (NMOSD).

Cladribine ODF can be also used at oncological pathology including first- and second-line treatment for symptomatic hairy cell leukemia and for B-cell chronic lymphocytic leukaemia, as well as cutaneous T-cell lymphoma, acute myeloid leukemia (AML), chronic lymphocytic leukemia (CLL), non-Hodgkin's lymphoma (NHL), autoimmune hemolytic anemia, mycosis fungoides, and Sezary syndrome.

The sublingual application of cladribine with the ODF technology offers the possibility to increase the bioavailability by circumventing the stomach degradation and absorption limitations in intestinum and therefore lowering the applied dosage of the drug. Therefore, the actual dosage of cladribine in the orodispersible film might be lower than the dosage applied in a conventional oral formulation.

According to another aspect, the present invention also relates to a transmucosal delivery composition in form of a sublingual orally dispersible film comprising cladribine as active ingredient for the treatment of neurodegenerative diseases with autoimmune origin, preferably multiple sclerosis (MS), myasthenia gravis (MG), or neuromyelitis optica spectrum disorders (NMOSD).

Cladribine is contained in the composition as a cladribine-cyclodextrin complex. This cladribine-cyclodextrin complex is present in the composition in a form mixed with at least two hydrophilic polymers which represent at least 20% w/w, more preferred at least 25% w/w, of the total composition. The at least two hydrophilic polymers comprise at least one structure forming polymer and at least one binding and/or intercalating polymer, wherein the at least one structure forming polymer is selected from starch polymers and wherein the at least one binding and/or intercalating polymer is pullulan.

As also demonstrated in the examples, compositions of the cladribine-cyclodextrin complex mixed with at least two hydrophilic polymers which represent at least 20% (w/w), preferably at least 21% (w/w) already provide good results homogeneity of the films and show very good disintegration times. In certain even more preferred embodiments, at least 22% (w/w), preferably at least 23% (w/w), especially least 25% (w/w), of the at least two hydrophilic polymers are foreseen in the total composition so as to obtain even more improved film preparations with regard to homogeneity, disintegration times.

The at least two hydrophilic polymers comprise at least one structure forming polymer and at least one binding and/or intercalating polymer. According to a preferred embodiment, the cyclodextrin is hydroxypropyl-β-cyclodextrin, γ-cyclodextrin, hydroxypropyl-γ-cyclodextrin, dimethyl-β-cyclodextrin, randomly methylated β-cyclodextrin, carboxymethyl-β-cyclodextrin or sulfobutyl-β-cyclodextrin. According to a preferred embodiment, the cladribine-cyclodextrin complex is comprising cladribine and cyclodextrin with a molar ratio from 1:1 to 1:9, preferably from 1:1 to 1:3.

The invention is further illustrated by the following examples and the figures, yet without being limited thereto.

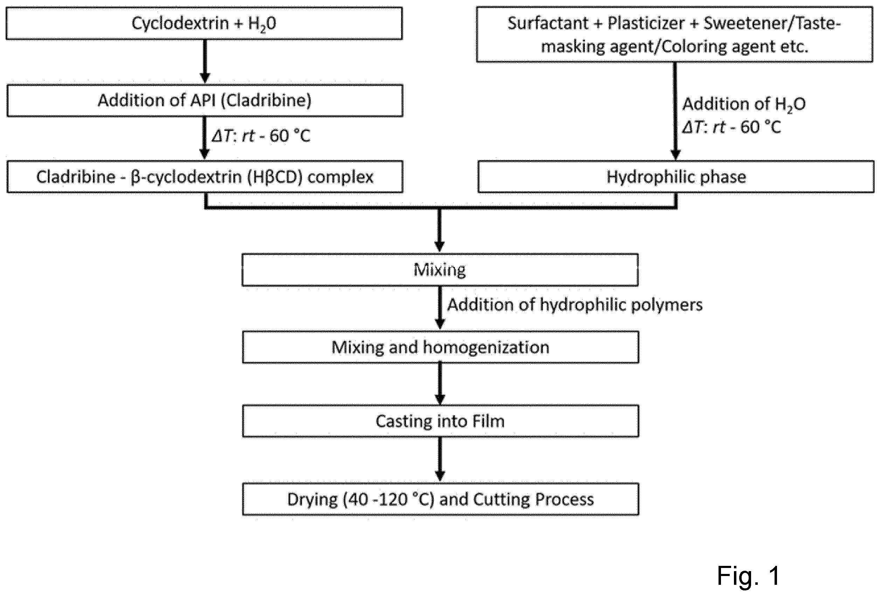

**Figure 1****.** Block diagram showing the manufacturing procedure of the orally disintegrating film according to an embodiment of this invention.

### Examples:

### General Procedure for making orally disintegrating thin films

The general procedure for the manufacturing of the orally disintegrating film composition is schematically illustrated in **Figure 1****.**

First, the cladribine-2-hydroxypropyl-β-cyclodextrin complex of cladribine with the *β-*cyclodextrin in water is formed upon mixing in a reaction vessel equipped with overhead stirrer. The mixture is stirred at room temperature or elevated temperatures until the active ingredient is homogenously suspended. In another reaction vessel equipped with an overhead stirrer, the surfactants (e.g. soybean lecithin with at least 70% phosphatidylcholine, such as Lipoid S75), the plasticizer (e.g. glycerol and/or triacetin) and the water-soluble additives were mixed in water at a minimum of 30 °C until all components are homogeneously dispersed. The previously prepared suspension containing the complex of cladribine and Kleptose HPB were added and the mixture was stirred with 200 - 1500 rpm) at a minimum of 30 °C for at least 15 min. After that the hydrophilic polymer or the mixture of hydrophilic polymers (e.g. modified starch and pullulan in the respective ratios) are added and the mixture is homogenized by stirring with 200 - 1000 rpm at a minimum of 30 °C for at least 15 min. The resulting dispersion is optionally degassed by stirring at room temperature or at 25 - 50 °C for 1-24 h or by stirring briefly under reduced pressure.

After that, a sufficient amount of water is added to exactly adjust the desired solid content (20-80 wt% w/w) and viscosity (100-10000 mPa.s (cP)) of the final coating mass. Using a mechanical casting apparatus equipped with doctor blade assembly, the stable coating mass is cast onto a substrate at a constant gap width, at the rate of 0.05-1.5 m/min, followed by drying at 40-100 °C using a conventional heating system or an IR-drying apparatus. With this drying process sufficient amounts of water in the composition are removed to produce a uniform thin film having an approximate thickness in the range of 20 to 500 microns. The film was subsequently cut into film strips of the desired size and shape either by carefully cutting the laminates with a knife/scalpel or by using a punching machine or by using a LASER cutting process.

Using the above-described manufacturing process, an oral disintegrating thin film containing cladribine in a cladribine-2-hydroxypropyl-β-cyclodextrin complex is produced. The active ingredient is uniformly distributed and the mechanical properties (dissolution, disintegration, elasticity etc.) of the film can be adjusted by the formulation and manufacturing process. This general manufacturing procedure ensures a very uniform distribution of active ingredient potency across the film surface, because the active ingredient is uniformly distributed within the polymeric matrix. Thus, the thin film will have a low relative standard deviation (RSD) of the concentration of active ingredient, resulting in more accurate dosing and thereby resulting in more consistent therapeutic efficacy, as compared to film strips of the prior art.

### Example 1: Compositions and method of making an oral disintegrating film containing cladribine

In a 100 mL reaction vessel equipped with an overhead stirrer, 15.38 g Kleptose HPB were suspended in 23,08 g deionized water at 300 rpm at room temperature. After that, 1.00 g cladribine was added and the mixture was stirred with 300 rpm at 50 °C for 3 h.

In another 250 mL reaction vessel equipped with an overhead stirrer 4.66 g glycerol and 0.26 g Lipoid S75 were suspended in 38.0 g water. The previously prepared API-suspension containing cladribine and Kleptose HPB was added and the mixture was stirred with a rate of 500 rpm at 50 °C for 60 min. Subsequently, 2.90 g Pullulan and 6.56 g Lycaot NG 720 were added and the coating mass was stirred with a rate of 500 rpm at 50 °C for 60 min. The solid content of the coating mass is adjusted zo 33.5% by addition of water to achieve the desired viscosity of the coating mass. After that, the stirring speed was reduced to 100 rpm and the coating mass was stirred until coating (ca. 1-4 h).

Using a mechanical casting apparatus equipped with doctor blade assembly and convection heating chamber, the stable dispersion is cast onto a PET substrate at the rate of 0.12 m/min and a gap width of 100 - 300 µm, followed by drying at 70-90 °C. With this coating procedure a thin film with uniform thickness and dry coating weight was produced.

**Table 1. Compositions of the orally disintegrating thin film containing cladribine.**

| Example 1 | |
|---|---|
| Component | wt% in dry mass |
| Cladribine | 3.25 |
| Kleptose HPB | 50.0 |
| Lipoid S75 | 0.85 |
| Lycoat NG 720 | 21.32 |
| Pullulan | 9.43 |
| Glycerol | 15.15 |
| Total | 100 |

The disintegration time of the ODFs of this example, determined by the method described above (see page 9), was between 120 - 180s.

### Example 2: Orally disintegrating film with a different molar ratio of cladribine and β-cyclodextrin

Example 2 is a composition of an orally disintegrating film with a different ratio of cladribine and *β*-cyclodextrin (molar ratio of cladribine : *β*-cyclodextrin 1:2). The formulation of Example 2 is summarized in Table 2. The film is prepared according to the general method described above and the method described in Example 1.

**Table 2. Compositions of the orally disintegrating thin film with a different molar ratio of cladribine and β-cyclodextrin.**

| Example 2 | |
|---|---|
| Component | wt% in dry mass |
| Cladribine | 3.25 |
| Kleptose HPB | 34.13 |
| Lipoid S75 | 0.85 |
| Lycoat NG 720 | 28.70 |
| Pullulan | 12.70 |
| Glycerol | 20.37 |
| Total | 100 |

The disintegration time of the ODFs of this example, determined by the method described above (see page 9), was between 120 - 180s.

### Example 3: Orally disintegrating film with a different ratio of modified starch and pullulan

Example 3 is a composition of an orally disintegrating film with a different ratio of modified starch and pullulan (50:50). The formulation of Example 3 is summarized in Table 3. The film is prepared according to the general method described above and the method described in Example 1.

**Table 3. Compositions of the orally disintegrating thin film with a different ratio of modified starch and Pullulan**

| Example 3 | |
|---|---|
| Component | wt% in dry mass |
| Cladribine | 3.25 |
| Kleptose HPB | 50.0 |
| Lipoid S75 | 0.85 |
| Lycoat NG 720 | 15.38 |
| Pullulan | 15.38 |
| Glycerol | 15.14 |
| Total | 100 |

The disintegration time of the ODFs of this example, determined by the method described above (see page 9), was between 120 - 180s.

### Example 4: Orally disintegrating film with a total amount of the two hydrophilic polymers of 23.4% (exemplified by modified starch and pullulan

Example 4 is a composition of an orally disintegrating film with a lower total amount of the polymers, modified starch and pullulan. The total combined amount (wt% in dry mass) of the polymers is in this Example 23.4%. The formulation of Example 4 is summarized in Table 4. The film is prepared according to the general method described above and the method described in Example 1.

The disintegration time of the ODFs of this example, determined by the method described above (see page 9), was below 30s.

**Table 4. Compositions of the orally disintegrating thin film with a combined amount of the polymers of 23.4%**

| Example 4 | |
|---|---|
| Component | wt% in dry mass |
| Cladribine | 4.25 |
| Kleptose HPB | 51.06 |
| Lipoid S75 | 2.13 |
| Lycoat RS 720 | 12.77 |
| Pullulan | 10.64 |
| Glycerol | 17.02 |
| Triacetin | 2.13 |
| Total | 100 |

### Example 5: Orally disintegrating film with lower total amount of the two hydrophilic polymers of 21.3% (exemplified by modified starch and pullulan)

Example 5 is a composition of an orally disintegrating film with a lower total amount of the polymers, modified starch and pullulan. The total combined amount (wt% in dry mass) of the polymers is in this Example 21.3%. The formulation of Example 5 is summarized in Table 5. The film is prepared according to the general method described above and the method described in Example 1.

The disintegration time of the ODFs of this example, determined by the method described above (see page 9), was below 30s.

**Table 5. Compositions of the orally disintegrating thin film with a combined amount of the polymers of 21.3%**

| Example 5 | |
|---|---|
| Component | wt% in dry mass |
| Cladribine | 4.25 |
| Kleptose HPB | 51.06 |
| Lipoid S75 | 2.13 |
| Lycoat RS 720 | 10.64 |
| Pullulan | 10.64 |
| Glycerol | 21.28 |
| Total | 100 |

### Comparative Example: Orally disintegrating film with an amount of the at least two hydrophilic polymers (exemplified by modified starch and pullulan) of below 20 % (w/w)

A formulation of 9.57% modified starch and 9.57% Pullulan (total amount of polymers 19.1%) did not result in a film with the desired properties, especially because of the low flexibility of the films obtained. The formulation of the Comparative Example is summarized in Table 6. The film was prepared according to the general method described above and the method described in Example 1.

**Table 6. Compositions of the orally disintegrating thin film with a combined amount of the polymers of 19.1%**

| Comparative Example | |
|---|---|
| Component | wt% in dry mass |
| Cladribine | 4.25 |
| Kleptose HPB | 51.06 |
| Lipoid S75 | 2.13 |
| Lycoat RS 720 | 9.57 |
| Pullulan | 9.57 |
| Glycerol | 23.42 |
| Total | 100 |

### Summary of the Examples

In conclusion, the studies of the above-described examples, which are manufactured according to the procedure described in the present invention, demonstrate that cladribine, in a cladribine-2-hydroxypropyl-β-cyclodextrin complex, can be incorporated and homogeneously distributed into a thin film. The active ingredient (cladribine) is homogenously distributed in a polymeric matrix within the thin film. With the procedure described in the present invention the production of ODFs containing cladribine as active ingredient is possible and also the properties of the ODFs can be modified by the formulation. This enables the manufacturing of novel sublingual dosage forms of cladribine with high accuracy and patient compliance.

The Examples 4 and 5 show that an orodispersible film can be produced with a total amount of polymers (combined amount of modified starch and pullulan) of respectively 23.4% and 21.3%, which is lower compared to Example 1, 2 and 3 (total amount of polymers 30.75%, 31,4% and 30.75%). The ODFs of these two examples showed very good homogeneity of the films and have very good disintegration properties. Thus, the disintegration time of the ODFs of these two examples, determined by the method described above (see page 9), is even below 30s, which is much lower compared to the disintegration time of the examples 1, 2 and 3 (disintegration time: 120 - 180 s).

However, following the general procedure described above, it was not possible with a formulation of 9.57% modified starch and 9.57% pullulan (total amount of polymers 19.1%) to achieve a film with the desired properties, because of the low viscosity of the coating mass and the low flexibility and homogeneity of the films (see: Comparative Example).

## Claims

1. A transmucosal delivery composition in form of a sublingual orally dispersible film comprising cladribine as active ingredient and comprising at least two hydrophilic polymers, wherein the at least two hydrophilic polymers represent at least 20 % w/w, preferably at least 25 % w/w, of the total composition, wherein cladribine is contained in the composition as a cladribine-cyclodextrin complex, wherein the at least two hydrophilic polymers comprise at least one structure forming polymer and at least one binding and/or intercalating polymer, wherein the at least one structure forming polymer is selected from starch polymers and wherein the at least one binding and/or intercalating polymer is pullulan.

2. The composition of claim 1, wherein the structure forming polymer has a molecular weight of above 100 000 g/mol.

3. The composition of claim 1 or 2, wherein the at least one binding and/or intercalating polymer has a molecular weight of 100 000 g/mol or below.

4. The composition according to claim 1 to 3 wherein the cyclodextrin is hydroxypropyl-β-cyclodextrin, γ-cyclodextrin, hydroxypropyl-γ-cyclodextrin, dimethyl-β-cyclodextrin, randomly methylated β-cyclodextrin, carboxymethyl-β-cyclodextrin or sulfobutyl-β-cyclodextrin.

5. The composition according to any one of claims 1 to 4, where cladribine-cyclodextrin complex is comprising cladribine and cyclodextrin with molar ratio from 1:1 to 1:9, preferably 1:1 to 1:3.

6. The composition according to any one of claims 1 to 5, comprising the active ingredient, the at least two hydrophilic polymers, and at least one surfactant and/or cosurfactant and at least one plasticizers and optionally further additives, such as taste masking agents, flavoring agents, solubilizers, thickeners, coloring agents, antioxidants, pH modifying agents, vitamins, or mixtures thereof.

7. The composition according to any one of claims 1 to 6, wherein the starch polymer is selected from a starch of acorn, arracacha, barley, beans, breadfruit, buckwheat, cassava, chickpea, chestnut, corn, lentil, millet, oat, pea, potato, rice, rye, sago, sorghum, sweet potato, tapioca, wheat and combinations thereof, a pregelatinized starch, a hydroxypropyl starch or a pregelatinized hydroxypropyl starch, a hydroxypropyl pea starch (non- or pregelatinized) said starches being pregelatinized, gelatinized, modified or unmodified.

8. The composition according to any one of claims 1 to 7, wherein the at least two hydrophilic polymers are used with a ratio of the starch polymers to pullulan of 95:5 to 5:95 by weight, preferably between 80:20 to 50:50 by weight, more preferably between 70:30 to 60:40 by weight.

9. The composition according to any one of claims 1 to 8, wherein cladribine is incorporated in a 2-hydroxypropyl-β-cyclodextrin complex, hydroxypropyl pea starch, pullulan, glycerol and, optionally, soybean lecithin with at least 70% phosphatidylcholine and/or triacetin.

10. The composition of any one of the preceding claims, wherein cladribine is present in amounts of 0.01% to 50% by weight of the total composition, preferably 0.1% to 20%, more preferably 1% to 10%.

11. The composition according to any one of preceding claims, wherein cladribine is the single effective ingredient, wherein the composition does not contain a further effective ingredient which is authorized to be administered to patients for the treatment of multiple sclerosis (MS), myasthenia gravis (MG), or neuromyelitis optica spectrum disorders (NMOSD).

12. A method for producing a transmucosal delivery composition in form of a sublingual orally dispersible film according to any one of claims 1 to 11, wherein cladribine or cladribine-cyclodextrin complex is mixed with film-forming polymers by stirring with at least 100 rpm for at least 10 min at a temperature of at least 30°C.

13. The composition of any one of claims 1 to 11, for use in the treatment of MS, MG, or NMOSD.

## Patentansprüche

1. Zusammensetzung zur transmukosalen Verabreichung in Form eines sublingualen, oral zersetzbaren Films, umfassend Cladribin als Wirkstoff und umfassend mindestens zwei hydrophile Polymere, wobei die mindestens zwei hydrophilen Polymere mindestens 20 Gew.-%, bevorzugt mindestens 25 Gew.-%, der Gesamtzusammensetzung ausmachen, wobei Cladribin in der Zusammensetzung als ein Cladribin-Cyclodextrin-Komplex enthalten ist, wobei die mindestens zwei hydrophilen Polymere mindestens ein strukturbildendes Polymer und mindestens ein bindendes und/oder interkalierendes Polymer umfassen, wobei das mindestens eine strukturbildende Polymer aus Stärke-Polymeren ausgewählt ist und wobei das mindestens eine bindende und/oder interkalierende Polymer Pullulan ist.

2. Zusammensetzung nach Anspruch 1, wobei das strukturbildende Polymer ein Molekulargewicht über 100 000 g/mol aufweist.

3. Zusammensetzung nach Anspruch 1 oder 2, wobei das mindestens eine bindende und/oder interkalierende Polymer ein Molekulargewicht von 100 000 g/mol oder weniger aufweist.

4. Zusammensetzung nach einem der Ansprüche 1 bis 3, wobei das Cyclodextrin Hydroxypropyl-β-Cyclodextrin, γ-Cyclodextrin, Hydroxypropyl-γ-Cyclodextrin, Dimethyl-β-Cyclodextrin, zufällig methyliertes β-Cyclodextrin, Carboxymethyl- β-Cyclodextrin oder Sulfobutyl-β-Cyclodextrin ist.

5. Zusammensetzung nach einem der Ansprüche 1 bis 4, wobei der Cladribin-Cyclodextrin-Komplex Cladribin und Cyclodextrin in einem molaren Verhältnis von 1:1 bis 1:9, bevorzugt 1:1 bis 1:3, umfasst.

6. Zusammensetzung nach einem der Ansprüche 1 bis 5, umfassend den Wirkstoff, die mindestens zwei hydrophilen Polymere und mindestens ein Tensid und/oder Co-Tensid und mindestens einen Weichmacher und gegebenenfalls weitere Zusatzstoffe, wie Geschmacksmaskierungsmittel, Geschmacksstoffe, Lösungsvermittler, Verdickungsmittel, Farbstoffe, Antioxidantien, pH-Modifizierungsmittel, Vitamine oder Mischungen davon.

7. Zusammensetzung nach einem der Ansprüche 1 bis 6, wobei das Stärke-Polymer ausgewählt ist aus einer Stärke aus Eichel, Arrakacha, Gerste, Bohnen, Brotfrucht, Buchweizen, Cassava, Kichererbse, Kastanie, Mais, Linse, Hirse, Hafer, Erbse, Kartoffel, Reis, Roggen, Sago, Sorghum, Süßkartoffel, Tapioka, Weizen und Kombinationen davon, einer vorverkleisterten Stärke, einer Hydroxypropylstärke oder einer vorverkleisterten Hydroxypropylstärke, einer Hydroxypropyl-Erbsenstärke (nicht- oder vorverkleistert), wobei die Stärken vorverkleistert, verkleistert, modifiziert oder unmodifiziert sind.

8. Zusammensetzung nach einem der Ansprüche 1 bis 7, wobei die mindestens zwei hydrophilen Polymere in einem Gewichtsverhältnis der Stärke-Polymere zu Pullulan von 95:5 bis 5:95, vorzugsweise von 80:20 bis 50:50, noch bevorzugter von 70:30 bis 60:40, verwendet werden.

9. Zusammensetzung nach einem der Ansprüche 1 bis 8, wobei Cladribin in einen 2-Hydroxypropyl-β-Cyclodextrin-Komplex, Hydroxypropyl-Erbsenstärke, Pullulan, Glycerin und gegebenenfalls Sojalecithin mit mindestens 70 % Phosphatidylcholin und/oder Triacetin eingearbeitet ist.

10. Zusammensetzung nach einem der vorhergehenden Ansprüche, wobei Cladribin in Mengen von 0,01 bis 50 Gew.-% der Gesamtzusammensetzung, bevorzugt 0,1 bis 20 %, noch bevorzugter 1 bis 10 %, vorhanden ist.

11. Zusammensetzung nach einem der vorhergehenden Ansprüche, wobei Cladribin der einzige wirksame Bestandteil ist, wobei die Zusammensetzung keinen weiteren wirksamen Bestandteil enthält, der zur Verabreichung an Patienten zur Behandlung von Multipler Sklerose (MS), Myasthenia gravis (MG) oder Neuromyelitis optica-Spektrumstörungen (NMOSD) zugelassen ist.

12. Verfahren zur Herstellung einer Zusammensetzung zur transmukosalen Verabreichung in Form eines sublingualen, oral zersetzbaren Films nach einem der Ansprüche 1 bis 11, wobei Cladribin oder Cladribin-Cyclodextrin-Komplex mit filmbildenden Polymeren durch Rühren mit mindestens 100 U/min für mindestens 10 min bei einer Temperatur von mindestens 30°C gemischt wird.

13. Die Zusammensetzung nach einem der Ansprüche 1 bis 11 zur Anwendung bei der Behandlung von MS, MG oder NMOSD.

## Revendications

1. Une composition de libération transmuqueuse sous forme d'un film oral dispersible sous-lingual comprenant la cladribine comme ingrédient actif et comprenant au moins deux polymères hydrophiles, **caractérisée en ce que** lesdits au moins deux polymères hydrophiles représentent au moins 20 % en poids, préférentiellement au moins 25 % en poids, de la composition totale, la cladribine étant présente dans la composition sous forme de complexe de cladribine-cyclodextrine, lesdits au moins deux polymères hydrophiles comprenant au moins un polymère structurant et au moins un polymère liant et/ou intercalant, le polymère structurant étant sélectionné parmi les polymères à base d'amidon et le polymère liant et/ou intercalant étant le pullulan.

2. La composition selon la revendication 1, **caractérisée en ce que** le polymère structurant a une masse moléculaire supérieure à 100 000 g/mol.

3. La composition selon la revendication 1 ou 2, **caractérisée en ce que** le polymère liant et/ou intercalant a une masse moléculaire inférieure ou égale à 100 000 g/mol.

4. La composition selon l'une quelconque des revendications 1 à 3, **caractérisée en ce que** la cyclodextrine est la hydroxypropyl-bêta-cyclodextrine, la gamma-cyclodextrine, la hydroxypropyl-gamma-cyclodextrine, la diméthyl-bêta-cyclodextrine, la bêta-cyclodextrine aléatoirement méthylée, la carboxyméthyl-bêta-cyclodextrine ou la sulfobutyl-bêta-cyclodextrine.

5. La composition selon l'une quelconque des revendications 1 à 4, **caractérisée en ce que** le complexe de cladribine-cyclodextrine comporte de la cladribine et de la cyclodextrine dans un rapport molaire de 1:1 à 1:9, préférentiellement de 1:1 à 1:3.

6. La composition selon l'une quelconque des revendications 1 à 5, comprenant l'ingrédient actif, lesdits au moins deux polymères hydrophiles, ainsi qu'au moins un tensioactif et/ou coten-sioactif et au moins un plastifiant, et éventuellement d'autres additifs tels que des agents masquants le goût, des arômes, des solubilisants, des épaississants, des colorants, des antioxydants, des agents modifiant le pH, des vitamines, ou des mélanges de ceux-ci.

7. La composition selon l'une quelconque des revendications 1 à 6, **caractérisée en ce que** le polymère à base d'amidon est sélectionné parmi un amidon de gland, d'arracacha, d'orge, de haricots, de breadfruit, de sarrasin, de manioc, de pois chiches, de châtaignier, de maïs, de lentilles, de mil, d'avoine, de pois, de pomme de terre, de riz, de seigle, de sagou, de sorgho, de patate douce, de tapioca, de blé et des combinaisons de ceux-ci, un amidon prégélifié, un amidon hydroxypropylé ou un amidon prégélifié hydroxypropylé, un amidon de pois hydroxypropylé (non ou prégélifié), ces amidons étant prégélifiés, gelifiés, modifiés ou non modifiés.

8. La composition selon l'une quelconque des revendications 1 à 7, **caractérisée en ce que** lesdits au moins deux polymères hydrophiles sont utilisés dans un rapport en poids des polymères à base d'amidon au pullulan de 95:5 à 5:95, préférentiellement de 80:20 à 50:50, plus préférentiellement de 70:30 à 60:40.

9. La composition selon l'une quelconque des revendications 1 à 8, **caractérisée en ce que** la cladribine est incorporée sous forme d'un complexe avec la hydroxypropyl-bêta-cyclodextrine, de l'amidon de pois hydroxypropylé, du pullulan, de la glycérine et, éventuellement, de la lécithine de soja avec au moins 70 % de phosphatidylcholine et/ou du triacétine.

10. La composition selon l'une quelconque des revendications précédentes, **caractérisée en ce que** la cladribine est présente en quantité de 0,01 % à 50 % en poids de la composition totale, préférentiellement de 0,1 % à 20 %, plus préférentiellement de 1 % à 10 %.

11. La composition selon l'une quelconque des revendications précédentes, **caractérisée en ce que** la cladribine est l'ingrédient actif unique, la composition ne comprenant pas un autre ingrédient actif autorisé à être administré aux patients pour le traitement de la sclérose en plaques (SEP), de la myasthénie grave (MG) ou du trouble du spectre de la neuromyélite optique (NMOSD).

12. Un procédé de production d'une composition de libération transmuqueuse sous forme d'un film oral dispersible sous-lingual selon l'une quelconque des revendications 1 à 11, **caractérisé en ce que** la cladribine ou le complexe de cladribine-cyclodextrine est mélangé avec des polymères formant un film par agitation à au moins 100 tr/min pendant au moins 10 minutes à une température d'au moins 30 °C.

13. La composition selon l'une quelconque des revendications 1 à 11, pour un usage dans le traitement de la SEP, de la MG ou du NMOSD.
